# EUROPEAN PATENT SPECIFICATION

(11) **EP 3 866 911 B1**
(45) Date of publication and mention of the grant of the patent: **10.01.2024**
(21) Application number: 19791207.4
(22) Date of filing: 18.10.2019
(51) Int. Cl.: A61N 1/05, A61N 1/375, A61N 1/39

(54) **IMPLANTABLE ELECTRODE LEAD WITH ACTIVE FIXATION**
IMPLANTIERBARE ELEKTRODENLEITUNG MIT AKTIVER FIXIERUNG
FIL D'ÉLECTRODE POUVANT ÊTRE IMPLANTÉ À FIXATION ACTIVE

(30) Priority: 19.10.2018 DE 102018126038
(43) Date of publication of application: 25.08.2021
(73) Proprietor: BIOTRONIK SE & Co. KG, 12359 Berlin (DE)
(72) Inventor: FRIEDRICH, Michael, 14532 Kleinmachnow (DE); KOLBERG, Gernot, 12161 Berlin (DE); WEISS, Ingo, 12435 Berlin (DE)
(74) Representative: Biotronik Corporate Services SE
(86) International application number: PCT/EP2019/078341
(87) International publication number: WO 2020/079213

(56) References cited:
- WO-A1-2014/182496
- WO-A1-2014/182603
- WO-A1-2016/073338
- WO-A1-2016/118712
- US-A1- 2018 256 904

## Description

The invention relates to an implantable extravascular electrode lead that can be connected to a pulse generator and that has an active device for anchoring in the tissue.

The vast majority of the defibrillators implanted today use implantable electrode leads that run via the vascular system into the heart of the patient in order to terminate critical cardiological conditions, such as e.g. tachycardias, there by intentionally delivering high-energy pulses (shocks) to the myocardium. On the other hand, one alternative design for an implantable defibrillator provides for placing the electrode lead within the body, but outside of the vascular system. In this case, the electrode leads are generally arranged subcutaneously or submuscularly in the extrathoracic region.

In order to permit optimal placement of the extravascular implantable defibrillator and its extravascular electrode leads, it is absolutely necessary that it be possible to attach the electrode leads to the tissue (e.g., muscle, fat, or fascia) to secure the electrode leads against unintentional displacement (dislocation) when they are implanted.

Once they have been inserted into the tissue through an incision, the previously known extravascular electrode leads for subcutaneous or submuscular implantation are generally attached in the tissue at the distal end of the electrode lead using a loop and/or using a circumferential fixing sleeve with a ligature by means of suturing.

However, it is a drawback especially of this type of fixation that an additional incision must be made in each case in order to be able to provide sutures at the relevant site. The patient receives undesired scars at the site of the additional incision. Furthermore, there is an additional risk of infection for each additional incision.

Furthermore, WO2014182603A1 discloses an implantable cardiac defibrillator (ICD) system including an ICD implanted subcutaneously in a patient, a defibrillation lead having a proximal portion coupled to the ICD and a distal portion having a defibrillation electrode configured to deliver a defibrillation or cardioversion shock to a heart of the patient, and a pacing lead that includes a distal portion having one or more electrodes and a proximal portion coupled to the ICD. The distal portion of the pacing lead is implanted at least partially along a posterior side of a sternum of the patient within the anterior mediastinum.

Further, D2 WO2016073338A1 describes a lead body having a defibrillation electrode positioned along a distal portion of the lead body. The defibrillation electrode includes a plurality of electrode segments spaced a distance apart from each other.

Further, WO2014182496A1 discloses anchoring mechanisms for an implantable electrical medical lead that is positioned within a substernal space. The anchoring mechanisms fixedly-position a distal portion of the lead, that is implanted in the substernal space.

Further, US20180256904A1 discloses a catheter for delivering an implantable medical lead to an implantation site near an ostium leading to a proximal region of a coronary sinus, wherein the catheter includes a distal end, a proximal end opposite the distal end, a tubular body extending between the distal and proximal ends, an atraumatic fixation structure defining a distal termination of the distal end, and a lead receiving lumen, wherein the atraumatic fixation structure is configured to enter the ostium and passively pivotally anchor with the proximal region of the coronary sinus. The lead receiving lumen extends along the tubular body from the proximal end to an opening defined in a side of the tubular body near the distal end and proximal the atraumatic fixation structure.

The aforesaid drawbacks therefore result in the object of creating an electrode lead and a system for inserting the electrode lead into the tissue, the lead being improved over the aforesaid drawbacks with respect to its anchoring to the tissue.

This object is attained using an implantable extravascular electrode lead according to the invention as defined in claim 1 and a system for inserting the electrode lead into the tissue according to claim 7.

The implantable extravascular electrode lead comprises an electrode body that has a proximal end and a distal end, wherein the implantable electrode lead is equipped in the region of the proximal end of the electrode lead body with a connecting device by means of which the electrode lead may be connected to an active device. The electrode lead furthermore comprises at least one electrical conductor arranged in the electrode lead body and at least one electrode pole, arranged in the region of the distal end and electrically connected to the at least one electrical conductor, for electrically contacting tissue surrounding the electrode lead when the electrode lead is implanted.

In the distal region of the electrode lead body or at the distal end of the electrode lead body, the electrode lead has a first fixing device by means of which the electrode lead may be fixed to patient body tissue in the extravascular region.

In this patent application, the distal region of the electrode lead body and even the electrode lead is considered that region of the electrode lead body or electrode lead that extends across maximally 30% of the length of the electrode lead body or electrode lead and that includes the distal end of the electrode lead body or electrode lead.

In one embodiment of the electrode lead, the fixing device extends lateral to the longitudinal axis of the electrode lead.

In one embodiment, the fixing device is mechanically actuatable. A cable, filament, wire, flexible member, stylet, or catheter may be used as the mechanical actuation means. So that the fixing device may be actuated by mean of the mechanical actuation means, the electrode lead may have a lumen, wherein the lumen extends from the proximal end of the electrode lead body to the distal end of the electrode lead body and runs within the electrode lead body. Depending on the application, the lumen may be open or closed at its distal end.

In another embodiment, the fixing device is electrically actuatable. For actuating the fixing device, the electrical energy may be transmitted to the fixing device, for example, from outside by means of an electrical lead running along the lead body of the electrode lead. Alternatively, the electrical energy may be transmitted to the fixing device from outside by means of electromagnetic waves.

In another embodiment, the fixing device is embodied in the shape of a helical element that extends about the electrode lead body. The helical element may have a pointed or sharp end. The helical element may furthermore be attached, with its end opposing the pointed tip, to the lead body. In another embodiment, the helical element may be attached to the lead body rotatable about the longitudinal axis of the lead body. In this case, the rotational movement required to screw the helical element into the tissue may be transmitted from the proximal end of the electrode lead to the distal end of the electrode lead, and there to the helical element, using a stylet inserted into a lumen of the electrode lead. The helical element in this exemplary embodiment is configured such that it may be screwed into the tissue in the one rotational direction and may be screwed out of the tissue again in the other rotational direction.

In another embodiment, a system comprising an implantable extravascular electrode lead and a catheter is suggested. In this system, highly elastic lamellae are arranged at the distal end of the electrode lead body of the electrode lead. The lamellae may assume a first loaded state and a second released state. In their loaded state, the tips of the lamellae point in the distal direction with respect to the electrode lead. In their released state, the tips of the lamellae point in the proximal direction with respect to the electrode lead. The lamellae assume their loaded state as long as the electrode lead and the lamellae are inserted completely into the catheter. If the catheter is retracted in the proximal direction with respect to the electrode lead, the lamellae shift to their released state as soon as the distal end of the catheter uncovers the lamellae. Due to their restoring force, which shifts the lamellae to their released state, in their released state the lamellae dig into the tissue, thus fixing the electrode lead to the tissue.

Particularly, one or a plurality of elastically deformable fixing elements are used as the fixing device. The fixing elements are free at their first end and with their second end are arranged on the electrode lead body. A fixing device comprises one or a plurality of fixing elements of the type just described. When there are a plurality of fixing elements, the latter are preferably arranged as a fixing device in a circle about the electrode lead body. A fixing device preferably comprises 2 to 16 fixing elements, more preferably 3 to 12 fixing elements, and more preferably 4 to 8 fixing elements. The fixing elements of the fixing device may be arranged individually on the electrode lead body. Alternatively, a carrier to which the fixing elements of a fixing device are attached and that then itself is arranged on the electrode lead body may be used.

Due to their elastic deformability the fixing elements may assume a first, loaded state and a second, released state. In their first, loaded state, the fixing elements have a largely longitudinally extended shape, while in their second, released state, the fixing elements assume their unrestricted configuration. The unrestricted configuration may preferably be a hook or claw shape. The hook shape is an angled structure, while the claw shape is formed in the shape of an arc or spiral arc. If the second end of the claw runs parallel to the electrode lead body, the arc or spiral segment of a released claw shape covers an angular range of 90° to 360°, preferably 90° to 270°. On the other hand, if the second end of the claw runs perpendicular to the electrode lead body, the arc or spiral segment of a released claw shape covers an angular range of 90° to 270°, preferably 90° to 180°.

An extravascular electrode lead must be fixed at its distal end or near its distal end to tissue so that the distal end of the electrode lead does not migrate over time towards the proximal end of the electrode lead due to body movement. For this it is advantageous when the free ends of the fixing elements of the fixing device arranged at the distal end of the electrode lead, when in their loaded state, point towards the distal end of the electrode lead, and move towards the proximal end when they shift to the released state. Due to their restoring force, which shifts the fixing elements, e.g. in the form of claws, to their released state, the fixing elements dig into or twist themselves into the tissue and, with their curved shape, fix the electrode lead to, e.g., muscle, fat, or fasciae.

Further, the extravascular electrode lead comprises a second fixing device being arranged on the electrode lead body between the proximal end of the electrode lead body and the fixing device (first fixing device) such that the electrode pole is disposed between the first fixing device and the second fixing device.

For delivering a shock, in their distal region extravascular electrode leads comprise large surface-area electrode poles that may preferably be embodied as helical electrodes, so-called shock coils. In addition to these large surface-area electrode poles, smaller electrode poles for recording cardiac signals may be arranged distal and/or proximal to these large surface-area electrode poles. These recording electrode poles are preferably embodied as annular electrode poles, also called ring electrodes. For reliable operation of an extravascular implantable defibrillator and its extravascular electrode leads, it is furthermore advantageous to secure the electrode leads against unintentional displacement (dislocation) in the region of the electrode poles when they are implanted. Fixing devices provided proximal and distal to the electrode poles are used to this end.

Further, the second fixing device comprises one or a plurality of fixing elements, wherein the fixing elements of the second fixing device likewise each have a free end, wherein the fixing elements may assume a first, loaded state and a second, released state, wherein in their first, loaded state the free ends of the fixing elements of the first fixing device point in the distal direction of the electrode lead, and in their first, loaded state the free ends of the fixing elements of the second fixing device point in the proximal direction of the electrode lead. In their second, released state, the free ends of the fixing elements of the second fixing device point in the distal direction of the electrode lead. Moreover, these described fixing elements of the second fixing device have the same properties as the previously described fixing elements of the first fixing device described previously.

Due to the mirror symmetrical arrangement of the first fixing device and second fixing device with respect to the longitudinal axis of the electrode lead, the region of the electrode lead arranged between the two fixing devices may be kept twisted and/or secured against unintentional displacements (dislocations) when implanted.

Alternatively, the second fixing device comprises one or a plurality of fixing elements, wherein the fixing elements of the second fixing device likewise each have a free end, wherein the fixing elements may assume a first, loaded state and a second, released state, wherein, in their first, loaded state, the free ends of the fixing elements of the first fixing device point in the distal direction of the electrode lead, and in their first, released state, as well as in their second, released state, the free ends of the fixing elements of the second fixing device point in the distal direction of the electrode lead. In this embodiment, the fixing elements of the second fixing device are preferably embodied curved or S-shaped. A curved fixing element in this context shall be construed to be an essentially planar, curved, or spiral-shaped fixing element that covers a circular arc of less than 90°, and wherein the one end of the arc-shaped fixing element is arranged on the electrode lead body and the second end of the arc-shaped fixing element terminates in a free end. An S-shaped fixing element shall be construed to mean that it comprises two arcs connected to one another that are curved in opposition to one another. The two arcs are arranged essentially in a planar manner, wherein the fixing element comprises a first arc that is arranged on the electrode lead body and a second arc that runs out in a free end.

The first arc can transition into the second arc or a linear intermediate piece may be arranged between the first arc and the second arc. The first arc and/or the second arc may cover an angle of 30° to 120°, preferably 45° to 110°, and more preferably 60° to 100°.

Due to the arc-shaped or S-shaped configuration of the fixing elements of the second fixing device, due to their restoring force, which shifts the fixing elements to their released state, the fixing elements press into the tissue and, with their arced shape, fix the electrode lead to, e.g., muscle, fat, or fascia to prevent migration of the second fixing device in the distal direction. Since, in their second, released state, the free ends of the fixing elements of the first fixing device point in the proximal direction and, in their second, released state, the free ends of the fixing elements of the second fixing devices point in the distal direction, the region of the electrode lead arranged between the two fixing device may be kept loaded and/or secured against unintentional displacement (dislocation) when implanted.

In another embodiment, if the second fixing device is formed from S-shaped fixing elements and claw-shaped fixing elements, and if the free ends of the S-shaped fixing elements, when in their released state, point in the distal direction of the electrode lead, and if the free ends of the claw-shaped fixing elements, when in their released state, point in the proximal direction of the electrode lead, then the second fixing device is secured against migration in the distal and proximal directions.

In an electrode lead with a first fixing device at the distal end having claw-shaped fixing elements in which, when the fixing elements are in the released state, the free ends point in the proximal direction of the electrode lead, and with a second fixing device that is arranged proximal to the first fixing device on the electrode lead body, and in which the second fixing device is equipped with S-shaped fixing elements, the free ends of which, when in the released state, point in the distal direction of the electrode lead, a catheter may be inserted from the proximal end towards the distal end via the electrode lead. If the connecting device of the electrode lead has a larger cross-section than the electrode lead body, during the production process the catheter may first be inserted via the electrode lead and then the plug may be mounted. If the catheter is moved from the proximal to the distal via the electrode lead, the fixing elements of the fixing devices are shifted from their second, released state to their first, loaded state.

In one embodiment, the second fixing device comprises 2 to 16 fixing elements, more preferably 3 to 12 fixing elements, and more preferably 4 to 8 fixing elements.

In another embodiment of the extravascular electrode lead, the fixing elements of the first fixing device and/or of the second fixing device are bioresorbable. A bioresorbable fixing element shall be construed to be a fixing element, the components of which may be broken down by the body. The bioresorbability has the effect that the fixing elements dissolve in a certain period of time. Because of this, the electrode lead may be explanted more easily.

In another embodiment of the extravascular electrode lead, the free ends of the fixing elements of the first fixing device and/or of the second fixing device are embodied as points or cutting edges. The points or cutting edges penetrate slightly into the tissue, thereby strengthening the fixation of the fixing device to the tissue.

In another embodiment of the extravascular electrode lead, the free ends of the fixing elements of the first fixing device and/or of the second fixing device are embodied as blunt, atraumatic ends. The atraumatic ends lead to less injury to the tissue. They lead the fixing device clawing or digging into the tissues. This leads to the tissue reacting less to the fixing device and therefore leads to less scarring
In another embodiment, the extravascular electrode lead comprises a lumen into which a stylet may be inserted. In this way the electrode lead may be advanced during the implantation process or, during withdrawal of implantation aids, such as e.g. delivery catheters, the electrode lead may be secured against inadvertent retraction in the proximal direction.

Moreover, a system for inserting an extravascular electrode lead into the tissue is suggested. The system comprises the implantable extravascular electrode lead described in the foregoing and a catheter having a catheter lumen for receiving the electrode lead, the electrode lead being arranged in the lumen of the catheter such that the fixing device is disposed within the lumen of the catheter.

When the electrode lead is inserted into the body, the catheter prevents direct contact between the fixing device and the surrounding tissue. This prevents damage to implantation material and injuries to body tissue caused by the fixing device. The catheter furthermore protects the fixing device from damage during implantation.

In another embodiment of the system for inserting an extravascular electrode lead, the electrode lead is arranged in the lumen of the catheter such that the fixing elements of the fixing device of the electrode lead assumes its first, loaded state.

In another embodiment of the system for inserting the electrode lead into the tissue, the electrode lead may be pushed out the catheter in the direction of its distal end such that when they exit from the lumen of the catheter, the fixing elements shift from their first, loaded state to their second, released state.

When the catheter is retracted in the proximal direction with respect to the electrode lead, the fixing elements of the fixing device shift to their second released state as soon as the distal end of the catheter uncovers the fixing elements. In their uncovered state, due to their restoring force, which shifts the fixing elements from their first, loaded state to their second, released state, the fixing elements uncovered by the catheter dig in or twist themselves into the tissue, thus fixing the electrode lead to the tissue.

Now, once the electrode lead has been inserted into an extravascular region of a patient, the fixing device is actively actuatable from outside the body for fixing the electrode lead to body tissue of the patient by means of the catheter.

In another embodiment, the system for inserting the electrode lead also comprises a stylet inserted into the lumen of the electrode lead. The stylet holds the electrode lead in its position when the catheter is to be retracted in the proximal direction of the electrode lead, so that, while the catheter is being retracted in the direction of the proximal end of the electrode lead, the electrode lead is not retracted together with the catheter, but instead the distal end of the electrode lead exits from the distal end of the catheter. Alternatively, during implantation the electrode lead with the stylet may be pushed out of the catheter in the distal direction of the electrode lead. This supports the fixing elements penetrating into the tissue and thus attains better fixation of the fixing device to the tissue. Improved fixation because of this has been seen in particular with fixing elements in the shape of claws.

In another embodiment, the system for inserting the electrode lead additionally comprises a slitter tool for cutting open the catheter while the catheter is being retracted in the proximal direction of the electrode lead. Since the electrode lead is equipped with a connecting device in the region of the proximal end of the electrode lead body, by means of which connecting device the electrode lead may be connected to an active device, a catheter may not be retracted in the proximal direction via the connecting device unless the cross-sectional area of the lumen of the catheter is greater than the cross-sectional area of the connecting device. Since the catheter is positioned close against the electrode lead so that the fixing elements of the fixing device may be held in their first, loaded state, this requires a connecting device that has essentially the cross-sectional area of the electrode lead body. As soon as the connecting device has a larger cross-sectional area than the electrode lead body, the catheter must be widened or opened in order to be able to be retracted over the connecting device. Using a slitting tool, the catheter may be cut open along its length while being retracted so that the electrode lead may be removed from the catheter through the slit thus created.

In another embodiment, the system for inserting the electrode lead comprises an electrode lead having two fixing devices. In this way the first fixing device and the second fixing device are uncovered one after the other when the catheter is retracted. In this way the region between the two fixing devices is fixed in a tensioned manner.

In another embodiment of the extravascular electrode lead, two opposing L-shaped hooks are arranged as fixing device at the distal end of the electrode lead body. The hooks have a pointed end. The end of the hook opposing the pointed end points into the lumen of the electrode lead body. In their first state, the hooks are extended out of the lumen of the electrode body, and, in their second state, they are pulled back into the lumen of the electrode lead body. In their first, extended state the two hooks are in a spread position and open at their pointed ends, and in their second, retracted state the hooks are pressed together and closed at their pointed end. When closed, the pointed ends of the hooks are oriented outward with respect to the longitudinal axis of the electrode lead body. The outwardly oriented pointed ends of the hooks fix the electrode lead in the tissue.

In another embodiment of the extravascular electrode lead, a helical screw element is arranged as the fixing device in the lumen of the electrode lead body in a rotatable manner and such that it may be screwed out. At its proximal end, the helical screw element has a guide with a slit in which a screw stylet may engage. The helical element may be screwed out of the lumen of the lead body by means of the screw stylet. In one embodiment of the helical screw element, the radius of the windings when the helical screw element is screwed out of the lumen of the lead body is greater in length than when it is screwed into the lumen, that is, when the helical screw element is retracted into the lumen. When screwed out, the pointed end of the helical screw element catches in the surrounding tissue, thus fixing the electrode lead to the tissue.

In another embodiment of the extravascular electrode lead, extendable claws are arranged in the lumen of the electrode lead body as the fixing device. At their first end, the claws are provided with a pointed tip and at their second end are attached to a guide device. The guide device may be advanced by means of a stylet inserted into the lumen of the electrode lead body from its first, retracted position in the distal direction of the electrode lead body into a second advanced position. When the guide device is in the retracted position, the claws are arranged within the lumen in their retracted position. The claws are in their loaded state when the claws are in the retracted position. When the claws are in the loaded state, the pointed ends of the claws point in the distal direction with respect to the electrode lead body. When the guide device is in the second, advanced position, the claws are in their extended position. In the extended position, the claws assume their released state. In their extended position, due to their restoring force, which shifts the claws to their released state, the claws dig into the tissue, thus fixing the electrode lead to the tissue.

In another embodiment of the extravascular electrode lead, the fixing device is realized in the form of self-deploying elements. To this end, the electrode lead body is surrounded by an outer shell that is connected to the lead body at the distal end of the electrode lead body. The outer shell has at least three parallel longitudinal slits running axially. The longitudinal slits are preferably provided equidistant with respect to the circumferential direction of the outer shell. The elements, each formed by two longitudinal slits in the outer shell, may assume two states. In their first state (starting position) they are straight, and in their second state (fixing position) they are curved. The elements may be shifted from their first, straight state to their second, curved state by moving the outer shell in the distal direction with respect to the electrode lead body. For this, for example the electrode lead body may be fixed to the proximal end and the outer shell may be advanced in the distal direction of the electrode lead. When advanced, the elements shift to their second, curved state. A detent device with which the outer shell may be held in its advanced state is preferably provided at the proximal end of the electrode lead. The detent device is preferably reversible, which means that it may be retracted in the distal direction again, releasable about the outer shell, in order to return the elements from their second curved state to their first straight state. This has the advantage that the electrode lead is moveable again and thus may be repositioned or explanted.

In another embodiment of the extravascular electrode lead, the fixing device is realized in the form of hooks deploying laterally out of the electrode lead body. The hooks in this case may assume a first side-by-side state and a second spread-apart state. The hooks are moveably connected at their first end to the electrode lead body in order to realize the movability required for the shift from the first state of the hooks to the second state of the hooks. The second, free end of each hook is freely moveable and may be side-by-side against the electrode lead body or spread apart from the electrode lead body. The free end of each hook may be equipped with a pointed tip. For moving the hooks, a lever that extends into the lumen of the electrode lead body may be provided on the side of the deployable hooks facing the lead body. To actuate the levers, a traction element that is connected to the levers runs within the lumen of the lead body. If the traction element is moved from its first, distal position into its second, proximal position, the deployable hooks shift from their first side-by-side state to their second spread-apart state. A tractive force act on the end of the traction element facing the proximal end of the electrode lead in order to shift the traction element from its first position to its second position. The traction element advantageously extends to the proximal end of the electrode lead.
In one preferred embodiment, the deployable hooks are arranged on the lead body such that the first end of each hook faces the distal end of the lead body and the second end of each hook faces the proximal end of the lead body.

In another embodiment of the extravascular electrode lead, the fixing device is embodied in the form of an elastic rod that can be bent at three points and that is arranged in the lumen of the electrode lead body. The bendable points of the rod may be called bend points. A bend point may be realized, for example, using a pinching of the rod or using an articulation, etc. Thus the rod comprises 4 segments, of which two segments (end segments) are each adj acent to only one bend point, and the other two segments (center segments) are each adjacent to two bend points. In this case the distal end of the rod facing the distal end of the electrode lead body is connected to the lead body. The proximal end of the rod opposing the distal end of the rod advantageously extends to the proximal end of the electrode lead. Moreover, the electrode lead body is provided with an axially running longitudinal slit at the height of the bend points of the rod.

The segments of the rod in a first state may assume a linear configuration; this means that the segments adjacent to a bend point assume an angle of approx. 180 degree to one another. In a second state, the segments of the rod may assume a V-shaped configuration; this means that the two center segments assume a triangular configuration, while the two end segments continue to follow the course of the electrode lead.

In a first state of the segments of the rod, all of the segments are disposed within the electrode lead body (deactivated fixing device). In the second state of the segments, the two center segments project from the axially running longitudinal slit of the electrode lead body (activated fixing device). The projecting center segments fix the electrode lead in the tissue. The segments may be moved from the first state of the segments to the second state of the segments by pushing the proximal end of the rod in the distal direction with respect to the electrode lead body.

In another embodiment of the extravascular electrode lead, the fixing device is provided in the form of a spiral band that may be pushed out of the electrode lead by a rotating mechanism arranged within the electrode lead. While it is being pushed out, the band, already pre-shaped in a spiral shape, is disposed in a spiral about the electrode lead body. While it is being screwed out, the tip of the band pushes into the tissue and in this way fixes the electrode lead to the tissue. The screwing mechanism may be actuated using a screw stylet inserted into the lumen of the electrode lead.

In another embodiment of the extravascular electrode lead, the fixing device is provided in the form of a balloon arranged at the distal end of the electrode lead body. For filling the balloon, the electrode lead comprises a lumen that runs from the proximal end of the electrode lead into the balloon. The balloon can be filled with a fluid through the lumen. The fluid can be added to the balloon with a syringe detachably arranged at the proximal end of the electrode lead, for example. In another embodiment, the fluid added to the balloon may cure in the balloon. Moreover, at its proximal end the lumen of the electrode lead may have a closing mechanism. The closing mechanism prevents fluid from exiting the balloon after the balloon has been filled with the fluid.

In another embodiment of the extravascular electrode lead, the fixing device is provided in the form of a distal region of the electrode lead body provided with apertures. A lumen is arranged within the electrode lead body to transport fluid from the proximal end of the electrode lead to the distal region of the electrode lead body. The apertures in the distal region of the electrode lead body connect the lumen running within the electrode lead body to the outside. The electrode lead can be filled with a fixing fluid at the proximal end. The fixing fluid added may exit from the electrode lead through the apertures in the distal region of the electrode lead body and then, in the implanted state, empties into the tissue. The fixing fluid is preferably a fluid that cures after flowing out of the apertures into the tissue. It is furthermore preferred that the fluid still has a certain flexibility after curing. The electrode lead is attached to the tissue by the fluid.

In another embodiment of the extravascular electrode lead, bent hooks are provided as the fixing device at the distal end of the electrode lead body and hook into the tissue, and thus fix the electrode lead to the tissue, when the electrode lead body is rotated, like a bayonet closure.

Additional features, advantages, and embodiments of the present invention shall be described in the following, referencing the figures.

Fig. 1 depicts a distal region of an electrode lead that is provided with a helical element, as fixing device, arranged about the lead body;
- Figs. 2a and 2b: depict a distal region of an electrode lead that is equipped with highly elastic lamellae;
- Figs. 2c through 2e: depict a distal region of an electrode lead that is equipped with highly elastic claws;
- Figs. 2f through 2h: depict a distal region of an electrode lead that is equipped with highly elastic claws and S-shaped fixing elements;
- Fig. 2i: depicts a part of an electrode lead body that is equipped with a fixing device that has both claws and S-shaped fixing elements;
- Figs. 3a and 3b: depict a distal region of an electrode lead having two hooks as the fixing device;
- Figs. 4a and 4b: depict a distal region of an electrode lead, out of which a helical element may be extended;
- Figs. 5a and 5b: depict a distal region of an electrode lead, claws being extendable out of the lumen thereof;
- Figs. 6a and 6b: depict a distal region of an electrode lead having self-deploying elements;
- Figs. 7a and 7b: depict a distal region of an electrode lead having laterally deployable hooks;
- Figs. 8a and 8b: depict a distal region of an electrode lead having an extendable bendable rod;
- Fig. 9: depicts a distal region of an electrode lead having a bayonet closure;
- Fig. 10: depicts a distal region of an electrode lead, out of which a spiral-shaped band may extend as a fixing device;
- Fig. 11a: depicts a distal region of an electrode lead having a balloon as fixing device; and,
- Fig. 11b: depicts a distal region of an electrode lead having apertures for adding a fixing fluid to the tissue.

The invention is explained in greater detail in the following using an implantable extravascular electrode lead. Such electrode leads are used in conjunction with subcutaneous defibrillators in which the stimulation electrode leads are not arranged in the vascular system, but rather are arranged subcutaneously or submuscularly in the tissue. Such electrode leads require a fixing device at their distal end in order to secure the electrode leads from unintentional dislocation when implanted by fixing them to the tissue.

A typical electrode lead for a subcutaneous defibrillator comprises an electrode lead body 10 having a proximal end 12 and a distal end 13. The proximal end 12 of the electrode lead body 10 is typically provided with a connecting device (not shown in the drawings) with which the electrode lead may be connected to a subcutaneous defibrillator. In its distal region the electrode lead furthermore has electrode poles 240 for electrically stimulating body tissue or for reading electrical signals from the body tissue. Provided within the lead body 10 are electrical leads (not shown) that extend from the proximal end of the lead body 10 to the distal region of the lead body 10 and that electrically connect the contacts in the connecting device to the electrode poles in order to transmit signals from the defibrillator to the electrode poles or to transmit signals recorded from the tissue back to the defibrillator.

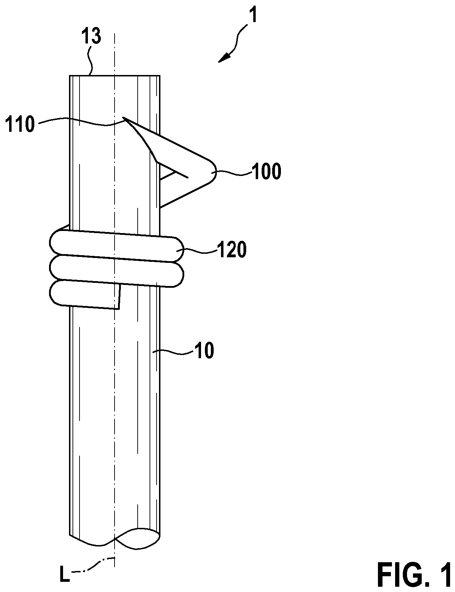

Figure 1 depicts a short distal end segment 1 of an electrode lead having a helical element 100 as fixing device to the tissue. The helical element is attached to a connecting point 120 on the electrode lead body 10 and is arranged such that it extends about the lead body 10. Moreover, the helix 100 comprises a pointed tip 110 with which the helical element may be screwed into the tissue by rotation on the electrode lead. In the example illustrated, the electrode lead must be rotated counterclockwise, from the perspective of the person performing the implantation, at the proximal end so that the helical element 100 screws into the tissue. Clockwise rotation permits the person performing the implantation to remove the electrode lead from the tissue again. If the electrode lead is not already fixed, clockwise rotation will not cause any damage.

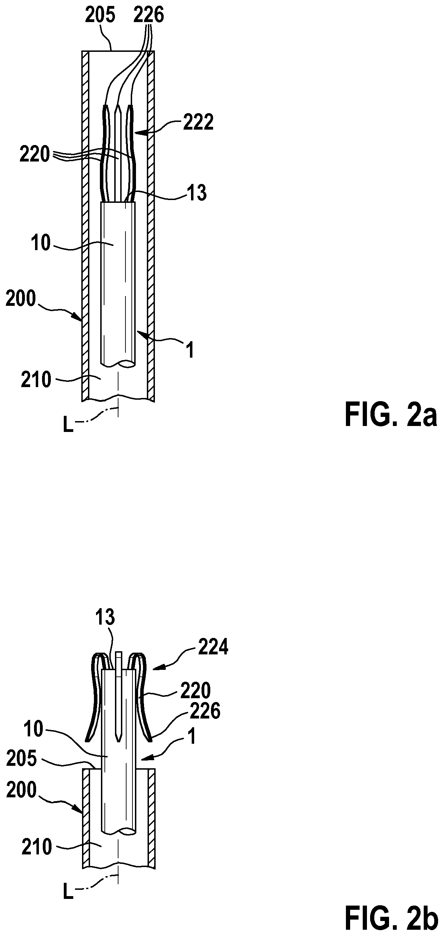

Figure 2a depicts the distal end segment 1 of an electrode lead inserted into the lumen 210 of a catheter 200. Attached to the distal end 13 of the lead body 10 are lamellae 220 that are in a loaded state 222 as long as the lamellae are completely enclosed by the catheter 200. If the catheter 200 is retracted in the proximal direction with respect to the lead body 10, as shown in Figure 2b, the lamellae 220 shift to their released state 224 as soon as the distal end 205 of the catheter 200 uncovers the lamellae 220. When uncovered, due to their restoring force, which shifts the lamellae to their released state 224, the lamellae 220 dig into the tissue with their pointed ends 226, thus fixing the electrode lead to the tissue.

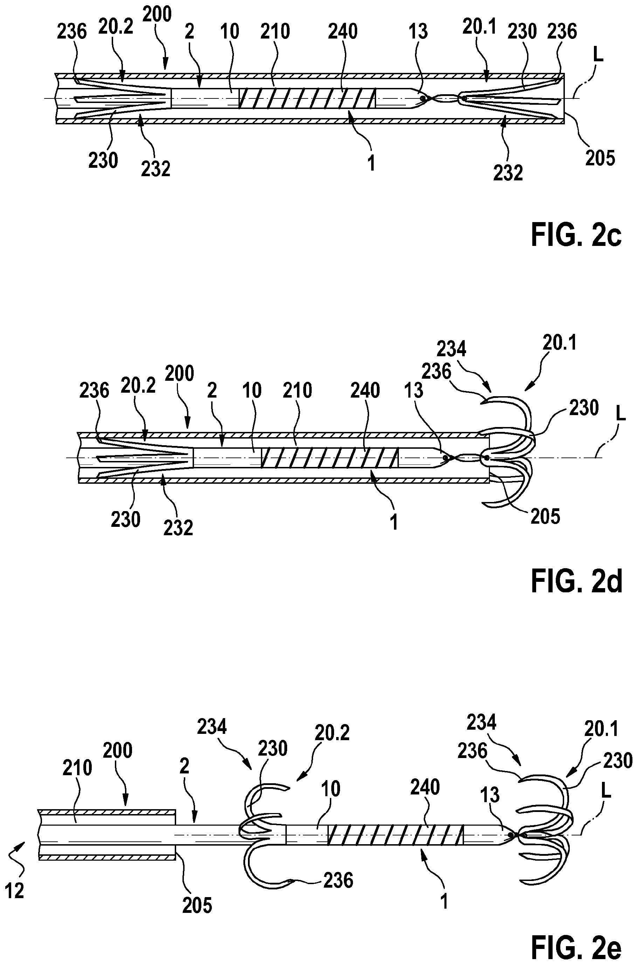

Figure 2c depicts the distal end segment 1 of an electrode lead 2 inserted into the lumen 210 of a catheter 200. Attached to the distal end 13 of the lead body 10 are claws 230 of a first fixing device 20.1 that are in a first, loaded state 232 as long as the claws 230 are completely enclosed by the catheter 200. If the catheter 200 is retracted in the proximal direction 12 with regard to the lead body 10, as shown in Figure 2d, the claws 230 of the first fixing device 20.1 shift into their second, released state 234 as soon as the distal end 205 of the catheter 200 uncovers the claws 230. When uncovered, due to their restoring force, which shifts the claws 230 from their first, loaded state 232 to their second, released state 234, the pointed ends 236 of the claws 230 dig into the tissue and thus fix the electrode lead 2 to the tissue.

If the catheter 200 is retracted further in the proximal direction 12 with respect to the lead body 10, as illustrated in Fig. 2e, the claws 230 of the second fixing device 20.2, which is arranged further proximally, shift to their second, released state 234 as soon as the distal end 205 of the catheter 200 uncovers the claws 230 of the second fixing device 20.2. When uncovered, due to their restoring force, which shifts the claws 230 from their first, loaded state 232 to their second, released state 234, the pointed ends 236 of the claws 230 of the second fixing device 20.2, also dig into the tissue, thus fixing the electrode lead 2 to the tissue at a second point.

A large surface-area electrode pole 240 for delivering shocks is arranged between the distally arranged first fixing device 20.1 and the further proximally arranged second fixing device 20.2 (see Figs. 2c through 2e). This large surface-area electrode pole 240 may be embodied, for example, as a coiled electrode (shock coil). Additional electrodes (not shown), e.g. in the form of ring electrodes, may be arranged between the electrode pole 240 and the first and/or the second fixing device 20.1, 20.2. These additional electrode poles may be used, e.g., for reading body signals.

For reliable operation of an extravascular implantable defibrillator (not shown) and its extravascular electrode leads 2, it is in addition advantageous to secure the electrode leads 2 in the region of the electrode pole 240 against unintentional dislocation when implanted. The fixing devices 20.1 and 20.2 provided proximal and distal to the electrode pole 240 serve this end. Due to the mirror-symmetrical arrangement of the first fixing device 20.1 and the second fixing device 20.2 with respect to an imaginary plane perpendicular to the longitudinal axis L of the electrode lead 2, the region between the two fixing devices 20.1, 20.2 may be held taut.

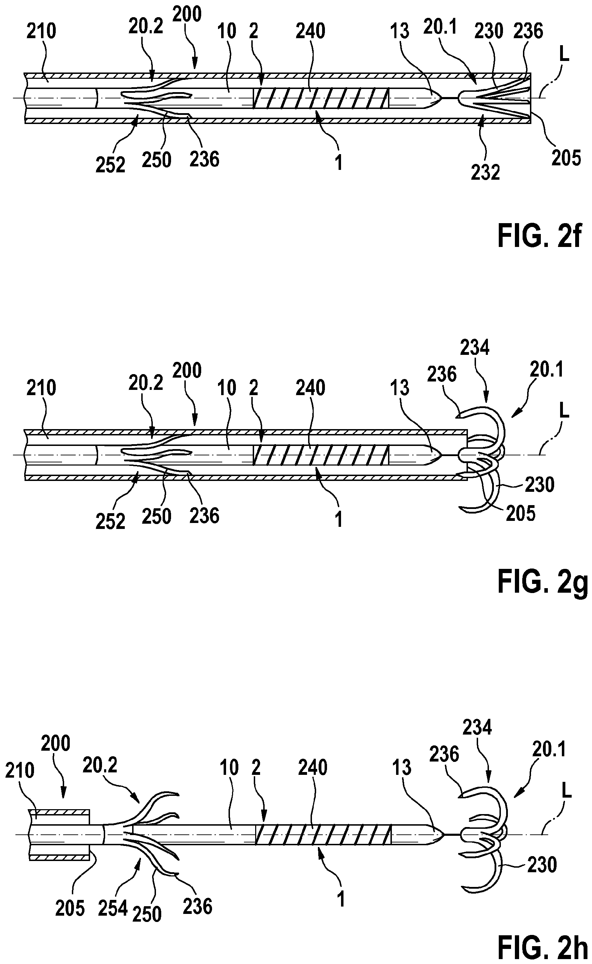

Figure 2f depicts the distal end section 1 of an electrode lead 2 inserted into the lumen 210 of a catheter 200. Claws 230 of a first fixing device 20.1 are attached to the distal end 13 of the lead body 10. Refer to the description for Figs. 2c and 2d with respect to the claws in Figs. 2f and 2g. In addition, a second fixing device 20.2 that has S-shaped fixing elements 250 with pointed ends 236 is arranged along the electrode lead body 10. Because of the catheter 200, the S-shaped fixing elements 250 are also in their first, loaded state 252. In contrast to Fig. 2c, the pointed ends 236 of the claws 230 and of the S-shaped fixing elements 250 in their loaded state point in the distal direction 13 of the electrode lead body 10. In addition, an electrode pole 240 is arranged between the first fixing device 20.1 and the second fixing device 20.2. Refer to Figs. 2c through 2e and the associated description with respect to the electrode pole 240 and other electrode poles.

If the catheter 200 is retracted further in the proximal direction 12 with respect to the electrode lead body 10, as illustrated in Fig. 2h, the S-shaped fixing elements 250 of the second fixing device 20.2 arranged further proximal shift to their second, released state 254 as soon as the distal end 205 of the catheter 200 uncovers the S-shaped fixing elements 250 of the second fixing device 20.2. When uncovered, due to their restoring force, which shifts the S-shaped fixing elements 250 from their first, loaded state 252 to their second, released state 254, the S-shaped fixing elements 250 of the second fixing device 20.2 press into the tissue, thus fixing the electrode lead 2 to a second point in the tissue.

The region between the two fixing devices 20.1, 20.2 can be held taut due to the pointed ends 236 of the claws 230 of the first fixing device 20.1 pointed in the proximal direction 12 of the electrode lead body 10 and the pointed ends 236 of the S-shaped fixing elements 250 of the second fixing device 20.2 pointing in the distal direction 13 of the electrode body 10.

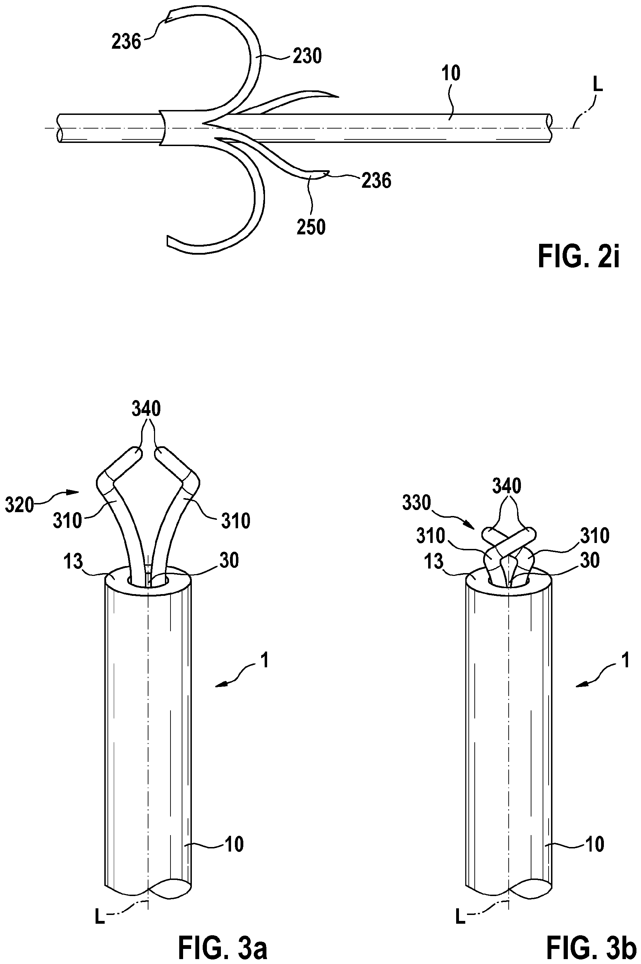

In Fig. 2i, the second fixing device 20.2 is formed from both S-shaped fixing elements 250 and from claw-shaped fixing elements 230. If this second fixing device 20.2 is arranged on the electrode lead body 10 such that in their released state 254 the pointed ends 236 of the S-shaped fixing elements 250 point in the distal direction 13 of the electrode lead body 10 and in their released state 234 the pointed ends 236 of the claw-shaped fixing elements 230 point in the proximal direction 12 of the electrode lead body 10, then the second fixing device 20.2 is secured against migration in the distal direction and in the proximal direction. Moreover, a catheter 200 may be placed over this fixing device 20.2 without any assisting devices, so that this catheter 200 shifts the claws 230 and the S-shaped fixing elements 250 from their released state 234, 254 to their loaded state 232, 252.

Figure 3a depicts another short distal end segment 1 of an electrode lead in which two opposing L-shaped hooks 310 are arranged as fixing device at the distal end 13 of the electrode lead body 10. The hooks 310 each have a pointed end 340. The end of a hook 310 opposing the pointed end 340 of the hook 310 points into the lumen 30 of the electrode lead body 10. In their first state the hooks 310 are extended out of the lumen 30 of the electrode lead body 10 (see Figure 3a) and in their second state they are pulled back into the lumen 30 of the electrode lead body 10 (see Figure 3b). In their first, extended state, the two hooks 310 are in a spread position 320 and open at their pointed ends 340. In their second, retracted state, the hooks 310 are in a pressed-together state 330 and closed at their pointed ends 340. In their closed state 330, the pointed ends 340 of the hooks 310 are oriented outward with respect to the longitudinal axis L of the electrode lead body 10 (see Figure 3b). The outwardly oriented pointed ends 340 of the hooks 310 fix the electrode lead in the tissue. A stylet, flexible rod, or cable may be used for extending the hooks 310 out of the lumen 30 of the electrode lead body 10 and for retracting the hooks 310 into the lumen 30 of the electrode lead body 10, for example.

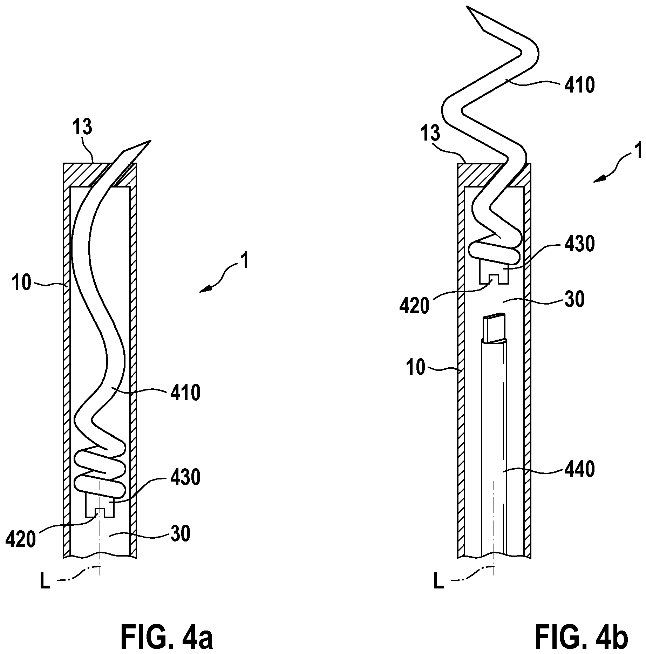

Figure 4a depicts another short distal end segment 1 of an electrode lead in which a helical screw element 410 is arranged as a fixing device in the lumen 30 of the electrode lead body 10 in a rotatable manner and such that it may be screwed out. At its proximal end, the helical screw element 410 has a guide 430 with a slit 420 in which a screw stylet 440 may engage. The helical screw element 410 may be screwed out of the lumen 30 of the lead body 10 by means of the screw stylet 440 (see Fig. 4b). In one embodiment of the helical screw element 410, the radius of the windings, when the helical screw element 410 is screwed out of the lumen 30 of the lead body 10, is greater in length than when the helical screw element 410 is screwed into the lumen 30, that is, retracted therein. When screwed out, the pointed end of the helical screw element 410 pricks into surrounding tissue, thus fixing the electrode lead to the tissue.

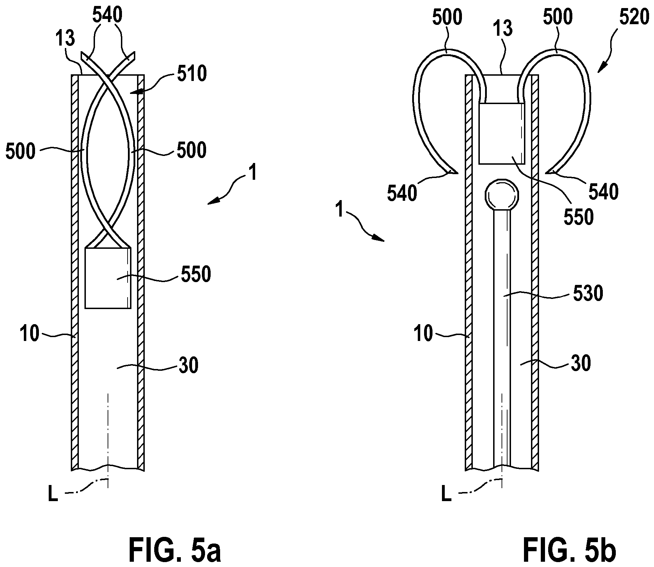

Figure 5a depicts another short distal end segment 1 of an electrode lead in which extendable claws 500 are arranged in the lumen 30 of the electrode lead body 10 as the fixing device. At their first end the claws 500 are provided with a tip 540, for example in the form of a sharpened end, and with their second end are attached to a guide device 550. The guide device 550 may be advanced towards the distal end 13 of the electrode lead body 10 from its first, retracted position (see Fig. 5a) into a second, advanced position (see Figure 5b) by means of a stylet 530 inserted into the lumen 30 of the electrode lead body 10. When the guide device 550 is in the retracted position, the claws 500 are arranged within the lumen 30 in the retracted position of the claws 500. The claws are in their loaded state 510 when the claws 500 are in the retracted position. When the claws 500 are in the loaded state 510, the pointed ends 540 of the claws 500 point in the distal direction with respect to the electrode lead body (see Figure 5a). When the guide device 550 is in the second advanced position, the claws 500 are in their extended position. In the extended position, the claws 500 assume their released state 520 (see Figure 5b). In their extended position, due to their restoring force, which shifts the claws 500 to their released state 520, the tips 540 of the claws 500 dig into the tissue, thus fixing the electrode lead to the tissue.

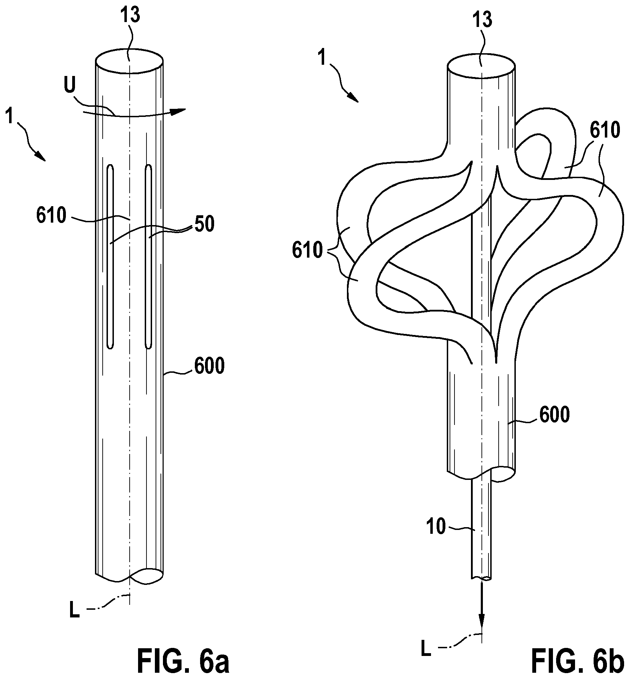

In the short distal end segment 1 of an electrode lead depicted in Figures 6a and 6b, the fixing device is realized in the form of self-deploying elements 610. To this end, the electrode lead body 10 is enclosed by an outer shell 600 that is connected to the lead body 10 at the distal end 13 of the electrode lead body 10. The outer shell 600 has at least three parallel longitudinal slits 50 running in the axial direction L. The longitudinal slits 50 are preferably provided equidistant with respect to the circumferential direction U of the outer shell 600. The elements 610, each formed by two longitudinal slits 50 in the outer shell 600, may assume two states. In their first state (starting position, see Figure 6a), they are straight, and in their second state (fixing position, see Figure 6b), they are curved. The elements 610 may be shifted from their first, straight state to their second, curved state by moving the outer shell 600 in the distal direction with respect to the electrode lead body 10. For this, for example the electrode lead body 10 may be fixed at the proximal end and the outer shell 600 may be advanced towards the distal end 13 of the electrode lead body 10. When advanced, the elements 610 shift to their second, curved state. A detent device with which the outer shell 600 may be held in its advanced state is preferably provided at the proximal end of the electrode lead. The detent device is preferably reversible, which means that it may be retracted again, releasable about the outer shell 600, in the proximal direction in order to return the elements 610 from their second curved state to their first straight state. This has the advantage that the electrode lead is moveable again and thus may be repositioned or explanted.

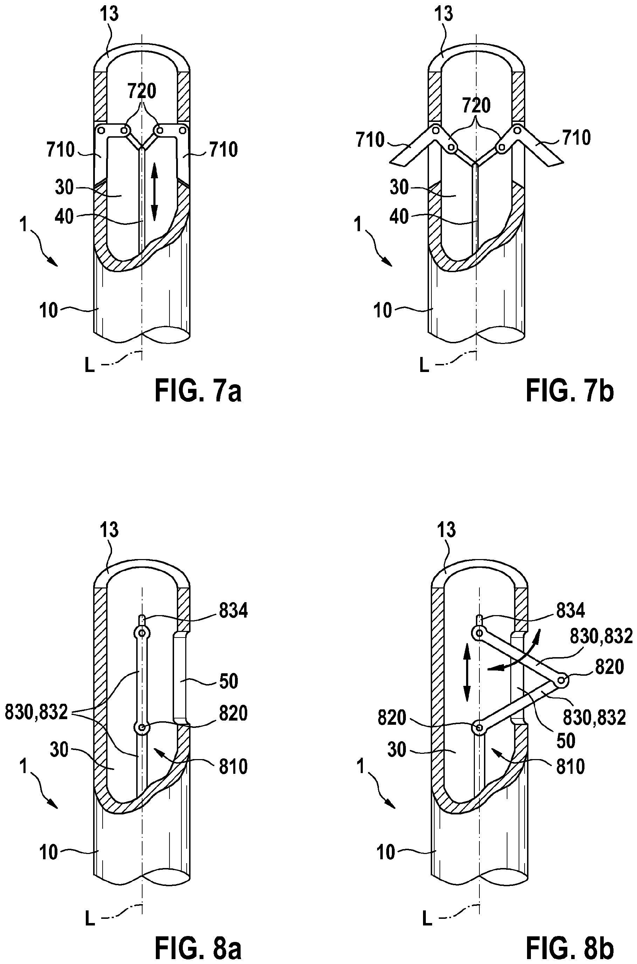

In the additional short distal end segment 1 of an electrode lead depicted in Figure 7a, the fixing device is realized in the form of hooks 710 deploying laterally out of the electrode lead body 10. The deploying hooks 710 in this case may assume a first side-by-side state (see Figure 7a) and a second spread-apart state (see Figure 7b). The hooks 710 are moveably connected at their first end to the electrode lead body 10 in order to realize the movability of the deploying hooks 710 required for the shift from the first state of the deploying hooks 710 to the second state of the deploying hooks 710. The second, free end of each hook 710 is freely moveable and may be side-by-side against the electrode lead body 10 or spread apart from the electrode lead body 10. The free end of each hook 710 may be equipped with a pointed tip. For moving the hooks 710, a lever 720 that extends into the lumen 30 of the electrode lead body 10 may be provided on the side of the deployable hooks facing the lead body 10. To actuate the levers 720, a traction element 40 that is connected to the levers 720 of the hooks 710 runs within the lumen 30 of the lead body 10. If the traction element 40 is moved from its first, distal position into its second, proximal position, the deploying hooks 710 shift from their first side-by-side state to their second spread-apart state. A tractive force must act on the end of the traction element 40 facing the proximal end of the electrode lead in order to shift the traction element 40 from its first position to its second position. The traction element 40 advantageously extends to the proximal end of the electrode lead. In one preferred embodiment, the deploying hooks 710 are arranged on the lead body 10 such that the first end of each hook faces the distal end of the lead body 13 and the second end of each hook faces the proximal end of the lead body (as illustrated in Figures 7a/b).

In the additional short distal end segment 1 of an electrode lead depicted in Figure 8a, the fixing device is embodied in the form of an elastic rod 810 that can be bent at three points and that is arranged in the lumen 30 of the electrode lead body 10. The bendable points 820 of the rod may be called bend points 820. A bend point 820 may be realized, for example, using a pinching of the rod or using an articulation, etc. Thus the rod 810 comprises 4 segments 830, of which two segments 830 (end segments 834) are each adjacent to only one bend point and the other two segments 830 (center segments 832) are each adjacent to two bend points 820. In this case the distal end 13 of the rod 810 facing the distal end of the electrode lead body 10 is connected to the lead body 10. The proximal end of the rod opposing the distal end of the rod advantageously extends to the proximal end of the electrode lead. Moreover, at the height of the bend points 820 of the rod 810, the electrode lead body 10 is provided with a longitudinal slit 50 running in the axial direct L.

The segments 830 of the rod 810 in a first state may assume a linear configuration (see Figure 8a); this means that the segments 830 adjacent to a bend point 820 assume an angle of approx. 180 degree to one another. In a second state, the segments 830 of the rod 810 may assume a V-shaped configuration (see Figure 8b); this means that the two center segments 832 assume a triangular configuration, while the two end segments 834 continue to follow the course of the electrode lead 10.

In the first state of the segments 830 of the rod 810, all of the segments 830 are disposed within the electrode lead body 10 (deactivated fixing device). In the second state of the segments 830, the center segments 832 project from the longitudinal slit 50 of the electrode lead body 10 that runs in the axial direction L (activated fixing device). The segments 830 may be moved from the first state of the segments 830 to the second state of the segments 830 by pushing the proximal end of the rod 810 in the distal direction with respect to the electrode lead body.

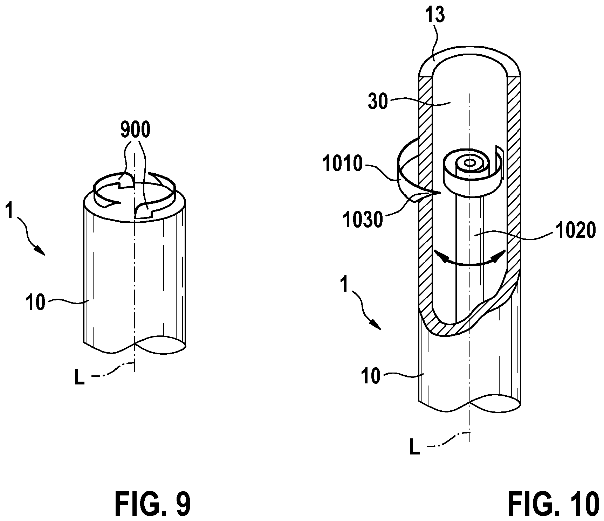

Figure 9 depicts another short distal end segment 1 of an electrode lead in which bent hooks 900 are provided as the fixing device at the distal end of the electrode lead body 10 and hook into the tissue when the electrode lead body 10 is rotated, like a bayonet closure.

In the additional short distal end segment 1 of an electrode lead depicted in Figure 10, the fixing device is provided in the form of a spiral band 1010 that may be pushed out of the electrode lead through a longitudinal slit 50 in the lead body 10 by a rotating mechanism arranged within the electrode lead body 10. While it is being pushed out, the band 1010, already pre-shaped in a spiral, is disposed in a spiral about the electrode lead body 10. While it is being screwed out, the tip 1030 of the band 1010 pushes into the tissue and in this way fixes the electrode lead to the tissue. The screwing mechanism may be actuated using a screw stylet 440 inserted into the lumen 30 of the electrode lead. The screw mechanism illustrated in Figure 10 comprises an axis 1020 onto which the band 1010 is wound.

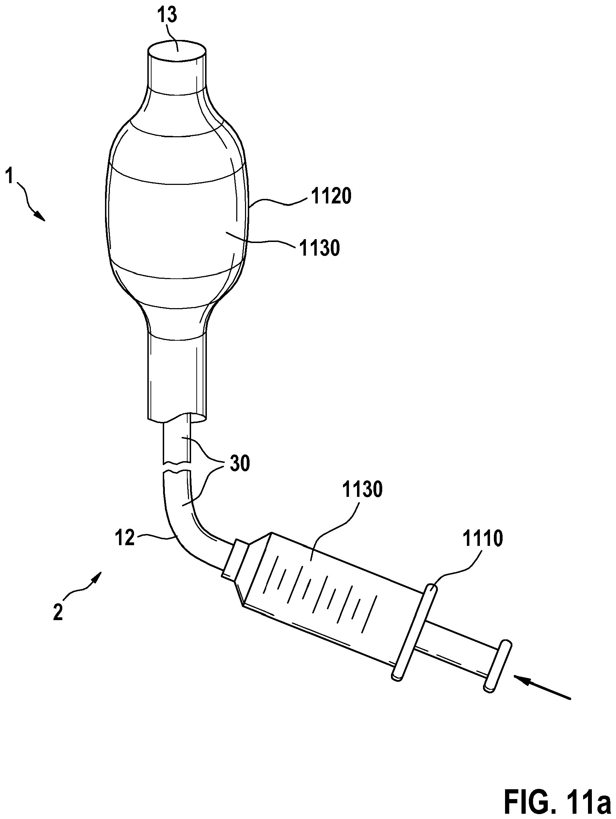

In the electrode lead 2 depicted in Figure 11a, the fixing device is provided in the form of a balloon 1120 arranged at the distal end 13 of the electrode lead body 10. For filling the balloon 1120, the electrode lead body 10 comprises a lumen 30 that runs from the proximal end 12 of the electrode lead into the balloon 1120. The balloon 1120 can be filled with a fluid 1130 through the lumen 30. The fluid 1130 can be added to the balloon 1120 with a syringe 1110 detachably arranged at the proximal end 12 of the electrode lead 2, for example. In another embodiment, the fluid 1130 added to the balloon 1120 may cure in the balloon 1120. Moreover, at its proximal end 12 the lumen 30 of the electrode lead 2 may have a closing mechanism. The closing mechanism prevents fluid 1130 from exiting the balloon 1120 after the balloon 1120 has been filled with the fluid 1130.

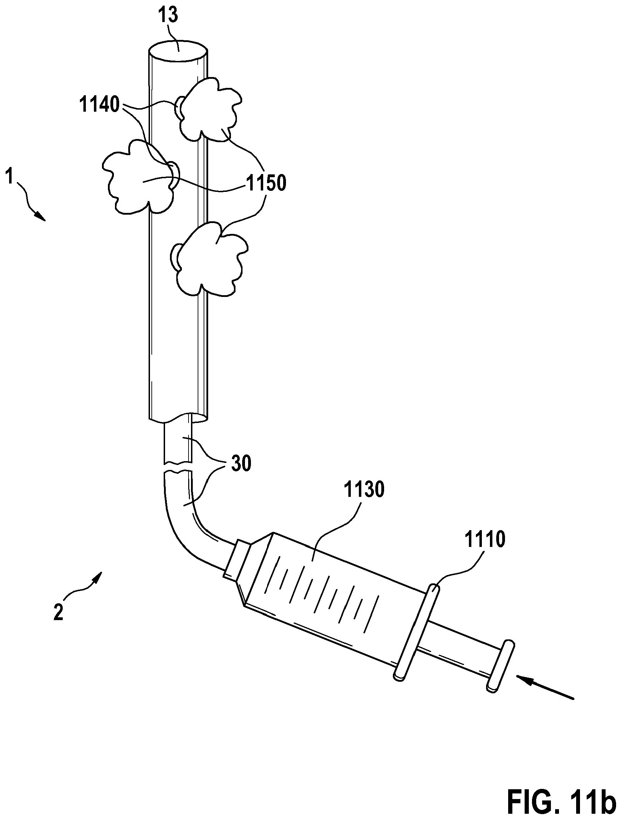

In the additional electrode lead 2 depicted in Figure 11b, the fixing device is provided in the form of a distal end segment 1 of the electrode lead 2 provided with apertures 1140. A lumen 30 is arranged within the electrode lead body 10 to transport fluid 1130 from the proximal end 12 of the electrode lead 2 to the distal end segment 1 of the electrode lead 2. The apertures 1140 at the distal end segment 1 of the electrode lead 2 connect the lumen 30 running within the electrode lead body 10 to the outside. The electrode lead 2 can be filled with a fixing fluid 1130 at the proximal end 12. The fixing fluid 1130 added may exit from the electrode lead 2 through the apertures 1140 in the distal end segment 1 of the electrode lead 2 and then, in the implanted state, empties into the tissue. The fixing fluid 1130 is preferably a fluid 1130 that cures 1150 after flowing out of the apertures 1140 into the tissue. It is furthermore preferred that after curing 1150 the fluid 1130 still has a certain flexibility. The electrode lead 2 is attached to the tissue by the fluid 1130.

## Claims

1. An implantable extravascular electrode lead (2), with an electrode lead body (10) that has a proximal end (12) and a distal end (13),
wherein the implantable electrode lead (2) is equipped in the region of the proximal end (12) of the electrode lead body (10) with a connecting device by means of which the electrode lead (2) may be connected to an active device,
with at least one electrical conductor arranged in the electrode lead body (10), and
with at least one electrode pole (240), arranged in the region of the distal end and electrically connected to the at least one electrical conductor, for electrically contacting tissue surrounding the electrode lead (2) when the electrode lead is implanted,
wherein, in the distal region (1) of the electrode lead body (10) or at the distal end (13) of the electrode lead body (10), the electrode lead (2) comprises a first fixing device (20.1) by means of which the electrode lead (2) may be fixed to patient body tissue in the extravascular region, wherein the first fixing device (20.1) comprises one or a plurality of fixing elements (230),
wherein the fixing elements (230) are configured to assume a first loaded state (232) and a second released state (234), wherein a second fixing device (20.2) may be arranged on the electrode lead body (10) between the proximal end (12) of the electrode lead body (10) and the first fixing device (20.1) such that the electrode pole (240) is disposed between the first fixing device (20.1) and the second fixing device (20.2),
**characterized in that**
like the first fixing device (20.1), the second fixing device (20.2) also comprises one or a plurality of fixing elements (230), wherein the fixing elements (230) of the first fixing device (20.1) and of the second fixing device (20.2) each have a free end (236), wherein the fixing elements (230) of the second fixing device (20.2) are also configured to assume a first, loaded state (232) and a second, released state (234), wherein in their first, loaded state (232) the free ends (236) of the fixing elements (230) of the first fixing device (20.1) point in the distal direction of the electrode lead (2), and in their first, loaded state (232) the free ends (236) of the fixing elements (230) of the second fixing device (20.2) point in the proximal direction of the electrode lead (2),
or
like the first fixing device (20.1), the second fixing device (20.2) also comprises one or a plurality of fixing elements (250), wherein the fixing elements (230, 250) of the first fixing device (20.1) and of the second fixing device (20.2) each have a free end (236), wherein the fixing elements (250) of the second fixing device (20.2) are also configured to assume
a first loaded state (252) and a second released state (254), wherein in their first loaded state (232) the free ends (236) of the fixing elements (230) of the first fixing device (20.1) point in the distal direction of the electrode lead (2), and in their first loaded state (252) and in their second, released state (254) the free ends (236) of the fixing elements (250) of the second fixing device (20.2) point in the distal direction of the electrode lead (2).

2. The implantable extravascular electrode lead (2) according to claim 1, wherein the fixing elements (230) are embodied in the shape of claws (230).

3. The implantable electrode lead (2) according to claims 1 through 2, wherein the first fixing device (20.1) comprises 2 to 16 fixing elements (230).

4. The implantable extravascular electrode lead (2) according to claims 1 through 3, wherein the second fixing device (20.2) comprises 2 to 16 fixing elements (230, 250).

5. The implantable extravascular electrode lead (2) according to claims 1 through 4, wherein the fixing elements (230, 250) are bioresorbable.

6. The implantable extravascular electrode lead (2) according to any of the preceding claims, wherein the electrode lead (2) comprises a lumen for a stylet.

7. A system for inserting the electrode lead into the tissue, comprising an implantable extravascular electrode lead (2) according to claims 1 through 6 and a catheter (200) having a catheter lumen (210) for receiving the electrode lead (2), wherein the electrode lead (2) is arranged in the lumen (210) of the catheter (200) such that the fixing device (20.1, 20.2) is disposed within the lumen (210) of the catheter (200).

8. The system for inserting the electrode lead into the tissue according to claim 7, wherein the electrode lead (2) is arranged in the lumen (210) of the catheter (200) such that the fixing elements (230, 250) of the fixing device (20.1, 20.2) assume their first, loaded state (232, 252).

9. The system for inserting the electrode lead into the tissue according to claim 7 or 8, wherein the electrode lead (2) may be moved out of the lumen (210) of the catheter (200) in the direction of its distal end (13) such that the fixing elements (230, 250), when exiting the lumen (210) of the catheter (200), shift from their first, loaded state (232, 252) to their second, released state (234, 254).

10. The system for inserting the electrode lead into the tissue according to any of claims 7 through 9, furthermore comprising a stylet inserted into the lumen of the electrode lead (2).

11. The system for inserting the electrode lead into the tissue according to any of claims 7 through 10, furthermore comprising a slitting tool for cutting open the catheter (200) while the catheter (200) is being retracted in the proximal direction (12) of the electrode lead (2).

## Patentansprüche

1. Eine implantierbare extravaskuläre Elektrodenleitung (2) mit einem Elektrodenleitungskörper (10), der ein proximales Ende (12) und ein distales Ende (13) aufweist,
wobei die implantierbare Elektrodenleitung (2) im Bereich des proximalen Endes (12) des Elektrodenleitungskörpers (10) mit einer Anschlussvorrichtung ausgestattet ist, mittels derer die Elektrodenleitung (2) mit einem aktiven Gerät verbunden werden kann, mit mindestens einem im Elektrodenleitungskörper (10) angeordneten elektrischen Leiter, und
mit mindestens einem im Bereich des distalen Endes angeordneten und mit dem mindestens einen elektrischen Leiter elektrisch verbundenen Elektrodenpol (240) zur elektrischen Kontaktierung von die Elektrodenleitung (2) umgebendem Gewebe bei der Implantation der Elektrodenleitung,
wobei die Elektrodenleitung (2) im distalen Bereich (1) des Elektrodenleitungskörpers (10) oder am distalen Ende (13) des Elektrodenleitungskörpers (10) eine erste Fixiereinrichtung (20.1) aufweist, mittels derer die Elektrodenleitung (2) im extravaskulären Bereich am Körpergewebe des Patienten fixierbar ist, wobei die erste Fixiereinrichtung (20.1) ein oder mehrere Fixierelemente (230) aufweist, wobei die Fixierelemente (230) eingerichtet sind, einen ersten belasteten Zustand (232) und einen zweiten gelösten Zustand (234) einzunehmen, wobei am Elektrodenleitungskörper (10) zwischen dem proximalen Ende (12) des Elektrodenleitungskörpers (10) und der ersten Fixiereinrichtung (20.1) eine zweite Fixiereinrichtung (20.2) angeordnet sein kann, so dass der Elektrodenpol (240) zwischen der ersten Fixiereinrichtung (20.1) und der zweiten Fixiereinrichtung (20.2) angeordnet ist,
**dadurch gekennzeichnet, dass**
wie die erste Fixiereinrichtung (20.1) auch die zweite Fixiereinrichtung (20.2) ein oder mehrere Fixierelemente (230) aufweist, wobei die Fixierelemente (230) der ersten Fixiereinrichtung (20.1) und der zweiten Fixiereinrichtung (20.2) jeweils ein freies Ende (236) aufweisen, wobei die Fixierelemente (230) der zweiten Fixiereinrichtung (20.2) dazu ausgebildet sind, einen ersten, belasteten Zustand (232) und einen zweiten, gelösten Zustand (234) einzunehmen, wobei die freien Enden (236) der Fixierelemente (230) der ersten Fixiereinrichtung (20.1) in ihrem ersten, belasteten Zustand (232) in die distale Richtung der Elektrodenleitung (2) zeigen, und die freien Enden (236) der Fixierelemente (230) der zweiten Fixiereinrichtung (20.2) in ihrem ersten, belasteten Zustand (232) in die proximale Richtung der Elektrodenleitung (2) zeigen,
oder
wie die erste Fixiereinrichtung (20.1) auch die zweite Fixiereinrichtung (20.2) ein oder mehrere Fixierelemente (250) aufweist, wobei die Fixierelemente (230, 250) der ersten Fixiereinrichtung (20.1) und der zweiten Fixiereinrichtung (20.2) jeweils ein freies Ende (236) aufweisen, wobei die Fixierelemente (250) der zweiten Fixiereinrichtung (20.2) dazu ausgebildet sind, einen ersten belasteten Zustand (252) und einen zweiten gelösten Zustand (254) einzunehmen, wobei die freien Enden (236) der Fixierelemente (230) der ersten Fixiereinrichtung (20.1) in ihrem ersten belasteten Zustand (232) in die distale Richtung der Elektrodenleitung (2) zeigen und die freien Enden (236) der Fixierelemente (250) der zweiten Fixiereinrichtung (20.2) in ihrem ersten belasteten Zustand (252) und in ihrem zweiten gelösten Zustand (254) in die distale Richtung der Elektrodenleitung (2) zeigen.

2. Implantierbare extravaskuläre Elektrodenleitung (2) nach Anspruch 1, wobei die Fixierelemente (230) in Form von Krallen (230) ausgebildet sind.

3. Implantierbare Elektrodenleitung (2) nach einem der Ansprüche 1 bis 2, wobei die erste Fixiereinrichtung (20.1) 2 bis 16 Fixierelemente (230) umfasst.

4. Implantierbare extravaskuläre Elektrodenleitung (2) nach einem der Ansprüche 1 bis 3, wobei die zweite Fixiereinrichtung (20.2) 2 bis 16 Fixierelemente (230, 250) umfasst.

5. Implantierbare extravaskuläre Elektrodenleitung (2) nach einem der Ansprüche 1 bis 4, wobei die Fixierelemente (230, 250) bioresorbierbar sind.

6. Implantierbare extravaskuläre Elektrodenleitung (2) nach einem der vorhergehenden Ansprüche, wobei die Elektrodenleitung (2) ein Lumen für ein Stilett aufweist.

7. System zum Einführen der Elektrodenleitung in das Gewebe, umfassend eine implantierbare extravaskuläre Elektrodenleitung (2) nach einem der Ansprüche 1 bis 6 und einen Katheter (200) mit einem Katheterlumen (210) zur Aufnahme der Elektrodenleitung (2), wobei die Elektrodenleitung (2) in dem Lumen (210) des Katheters (200) derart angeordnet ist, dass die Fixiereinrichtung (20.1, 20.2) innerhalb des Lumens (210) des Katheters (200) angeordnet ist.

8. System zum Einführen der Elektrodenleitung in das Gewebe nach Anspruch 7, wobei die Elektrodenleitung (2) so im Lumen (210) des Katheters (200) angeordnet ist, dass die Fixierelemente (230, 250) der Fixiereinrichtung (20.1, 20.2) ihren ersten, belasteten Zustand (232, 252) einnehmen.

9. System zum Einführen der Elektrodenleitung in das Gewebe nach Anspruch 7 oder 8, wobei die Elektrodenleitung (2) aus dem Lumen (210) des Katheters (200) in Richtung seines distalen Endes (13) herausbewegt werden kann, so dass die Fixierelemente (230, 250) beim Verlassen des Lumens (210) des Katheters (200) von ihrem ersten, belasteten Zustand (232, 252) in ihren zweiten, gelösten Zustand (234, 254) übergehen.

10. System zum Einführen der Elektrodenleitung in das Gewebe nach einem der Ansprüche 7 bis 9, das außerdem ein in das Lumen der Elektrodenleitung (2) eingeführtes Stilett umfasst.

11. System zum Einführen der Elektrodenleitung in das Gewebe nach einem der Ansprüche 7 bis 10, das außerdem ein Schlitzwerkzeug zum Aufschneiden des Katheters (200), während der Katheter (200) in die proximale Richtung (12) der Elektrodenleitung (2) zurückgezogen wird, umfasst.

## Revendications

1. Un fil d'électrode extravasculaire implantable (2), avec un corps de fil d'électrode (10) ayant une extrémité proximale (12) et une extrémité distale (13),
dans lequel le fil d'électrode implantable (2) est équipée, au niveau de l'extrémité proximale (12) du corps de fil d'électrode (10), d'un dispositif de connexion au moyen duquel le fil d'électrode (2) peut être connectée à un dispositif actif,
avec au moins un conducteur électrique disposé dans le corps de fil d'électrode (10), et avec au moins un pôle d'électrode (240), disposé dans la région de l'extrémité distale et connecté électriquement à au moins un conducteur électrique, pour établir un contact électrique avec le tissu entourant le fil d'électrode (2) lorsque le fil d'électrode est implantée,
dans lequel, dans la région distale (1) du corps de fil d'électrode (10) ou à l'extrémité distale (13) du corps de fil d'électrode (10), le fil d'électrode (2) comprend un premier dispositif de fixation (20.1) au moyen duquel le fil d'électrode (2) peut être fixée au tissu corporel du patient dans la région extravasculaire, dans lequel le premier dispositif de fixation (20.1) comprend un ou plusieurs éléments de fixation (230), dans lequel les éléments de fixation (230) sont configurés pour prendre un premier état chargé (232) et un second état relâché (234), (234), dans lequel un deuxième dispositif de fixation (20.2) peut être disposé sur le corps de fil l'électrode (10) entre l'extrémité proximale (12) du corps de fil l'électrode (10) et le premier dispositif de fixation (20.1) de sorte que le pôle de l'électrode (240) est disposé entre le premier dispositif de fixation (20.1) et le deuxième dispositif de fixation (20.2),
**caractérisé en ce que**
comme le premier dispositif de fixation (20.1), le deuxième dispositif de fixation (20.2) comprend également un ou plusieurs éléments de fixation (230), dans lequel les éléments de fixation (230) du premier dispositif de fixation (20.1) et du deuxième dispositif de fixation (20.2) ont chacun une extrémité libre (236), dans lequel les éléments de fixation (230) du deuxième dispositif de fixation (20.2) sont également configurés pour prendre un premier état chargé (232) et un deuxième état relâché (234), dans lequel dans leur premier état chargé (232), les extrémités libres (236) des éléments de fixation (230) du premier dispositif de fixation (20.1) pointent dans la direction distale du fil d'électrode (2), et dans leur premier état chargé (232), les extrémités libres (236) des éléments de fixation (230) du deuxième dispositif de fixation (20.2) pointent dans la direction proximale du fil d'électrode (2),
comme le premier dispositif de fixation (20.1), le deuxième dispositif de fixation (20.2) comprend également un ou plusieurs éléments de fixation (250), dans lequel les éléments de fixation (230, 250) du premier dispositif de fixation (20.1) et du deuxième dispositif de fixation (20.2) ont chacun une extrémité libre (236), dans lequel les éléments de fixation (250) du deuxième dispositif de fixation (20.2) sont également configurés pour prendre un premier état chargé (252) et un deuxième état relâché (254), dans lequel, dans leur premier état chargé (232), les extrémités libres (236) des éléments de fixation (230) du premier dispositif de fixation (20.1) pointent dans la direction distale du fil d'électrode (2), et dans leur premier état chargé (252) et dans leur deuxième état relâché (254), les extrémités libres (236) des éléments de fixation (250) du deuxième dispositif de fixation (20.2) pointent dans la direction distale du fil d'électrode (2).

2. Le fil d'électrode extravasculaire implantable (2) selon la revendication 1, dans lequel les éléments de fixation (230) ont la forme de griffes (230).

3. Le fil d'électrode implantable (2) selon les revendications 1 à 2, dans lequel le premier dispositif de fixation (20.1) comprend de 2 à 16 éléments de fixation (230).

4. Le fil d'électrode extravasculaire implantable (2) selon les revendications 1 à 3, dans lequel le deuxième dispositif de fixation (20.2) comprend 2 à 16 éléments de fixation (230, 250).

5. Le fil d'électrode extravasculaire implantable (2) selon les revendications 1 à 4, dans lequel les éléments de fixation (230, 250) sont biorésorbables.

6. Le fil d'électrode extravasculaire implantable (2) selon l'une des revendications précédentes, dans lequel le fil d'électrode (2) comprend une lumière pour un stylet.

7. Système pour insérer le fil d'électrode dans le tissu, comprenant un fil d'électrode extravasculaire implantable (2) selon les revendications 1 à 6 et un cathéter (200) ayant une lumière de cathéter (210) pour recevoir le fil d'électrode (2), dans lequel le fil d'électrode (2) est disposée dans la lumière (210) du cathéter (200) de telle sorte que le dispositif de fixation (20.1, 20.2) est disposé dans la lumière (210) du cathéter (200).

8. Système pour insérer le fil d'électrode dans le tissu selon la revendication 7, dans lequel le fil d'électrode (2) est disposée dans la lumière (210) du cathéter (200) de telle sorte que les éléments de fixation (230, 250) du dispositif de fixation (20.1, 20.2) prennent leur premier état chargé (232, 252).

9. Système pour insérer le fil d'électrode dans le tissu selon la revendication 7 ou 8, dans lequel le fil d'électrode (2) peut être déplacée hors de la lumière (210) du cathéter (200) en direction de son extrémité distale (13) de sorte que les éléments de fixation (230, 250), lorsqu'ils sortent de la lumière (210) du cathéter (200), passent de leur premier état de charge (232, 252) à leur deuxième état relâché (234, 254).

10. Système pour insérer le fil d'électrode dans le tissu selon l'une des revendications 7 à 9, comprenant en outre un stylet inséré dans la lumière du fil d'électrode (2).

11. Système pour insérer le fil d'électrode dans le tissu selon l'une des revendications 7 à 10, comprenant en outre un outil de découpe pour ouvrir le cathéter (200) pendant que le cathéter (200) est rétracté dans la direction proximale (12) du fil d'électrode (2).
